(19) Europäisches Patentamt
European Patent Office
Office européen des brevets

(11) Publication number : 0 420 121 B1

(12) EUROPEAN PATENT SPECIFICATION

(45) Date of publication of patent specification : 17.03.93 Bulletin 93/11

(51) Int. Cl.$^5$ : **A61K 37/02**, A61K 37/64

(21) Application number : 90118339.2

(22) Date of filing : 25.09.90

# (54) Antitumor composition.

(30) Priority : 27.09.89 JP 251534/89

(43) Date of publication of application : 03.04.91 Bulletin 91/14

(45) Publication of the grant of the patent : 17.03.93 Bulletin 93/11

(84) Designated Contracting States : AT BE CH DE DK ES FR GB GR IT LI LU NL SE

(56) References cited :
WO-A-89/00427
US-A- 3 984 569
BIOCHEMICAL AND BIOPHYSICAL RESEARCH COMMUNICATIONS, vol. 35, no. 5, 1969, New York, NY (US); R.VINCE et al., pp. 593-598*

(73) Proprietor : Senju Pharmaceutical Co., Ltd.
5-8, Hiranomachi 2-chome, Chuo-Ku
Osaka-shi, Osaka 541 (JP)

(72) Inventor : Ohmori, Shinji
14-5, Gion
Okayama-shi, Okayama 703 (JP)
Inventor : Ogata, Kazumi
B-701, 8, Kamishinden 4-chome
Toyonaka-shi, Osaka 565 (JP)
Inventor : Sakaue, Takahiro
1, Takadai 5-chome
Itami-shi, Hyogo 664 (JP)

(74) Representative : von Kreisler, Alek, Dipl.-Chem. et al
Patentanwälte Von Kreisler-Selting-Werner,
Deichmannhaus am Hauptbahnhof
W-5000 Köln 1 (DE)

EP 0 420 121 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an antitumor agent. More particularly, it relates to a useful antitumor composition which contains S-(α ,β - dicarboxyethyl)glutathione which is found in the mammalian body, or an ester derivatives thereof, either in the free form or in a pharmaceutically acceptable salt form.

Hitherto a number of agents, for example chemotherapeutic agents and immunotherapeutic agents, have been used in the treatment of malignant tumors. Howuever, none of them has been found to be fully satisfactory since they are insufficient in efficacy and/or produce severe adverse effects.

S-alkyl derivatives of glutathione and their glyoxalase I inhibiting effects are known from Biochem. Biophys. Res. Com., Vol. 35, No. 5, pp. 593-598 (1969). Furthermore, the use of S-alkyl-, S-acetyl-, and S-phospho - glutathione as tumor therapeutics is disclosed in W0-A-89/00427 as well as that reduced glutathione (S-unsubstituted) exhibits anti-melanoma activity in mice.

The present inventors made intensive investigation in search of compounds having good antitumor activity with low risks for adverse effects and, as a result found that S-(α ,β - dicarboxyethyl)glutathione, which is a substance present in the mammalian body, and ester derivatives thereof unexpectedly have very potent antitumor activity and at the same time have a high level of safety. The present invention has been accomplished on the basis of the above findings.

The invention provides the use of a compound of the formula

```
HOOC-CH-CH2CH2-CONH-CH-CONH-CH2-COOH
     |                 |
     NH2               CH2-S-CH-COOR
                            |
                            CH2-COOR
```

wherein two R's are the same or different and each is a hydrogen atom or a lower alkyl group having 1-10 carbon atoms or a pharmacologically acceptable salt thereof as an active ingredient for preparing a medicament for fighting hepatic cancer.

DETAILED DESCRIPTION OF THE INVENTION

Among the active ingredients to be used in the composition according to the invention, S-(α ,β -dicarboxyethyl)glutathione is a physiological substance discovered in the bovine crystalline lens by D. H. Calam and S. G. Waley [biochem. J., 86, 226 (1963)]. At present, however, only a little is known about its pharmacological features. Its use in cosmetic compositions for treating the hair is disclosed in US-A-3 984 569.

The present inventors have previously found that this compound has blood coagulation inhibiting and platelet aggregation inhibiting activities as well as antiinflammatory and/or antiallergic activity [Japanese Published Unexamined Patent Application (Kokai) No. 63-8337 and Japanese Patent Applications Nos. 1-79956 and 1-83484].

In the above formula, two R's are the same or different and each is a hydrogen atom or a lower alkyl group containing 1 to 10 carbon atoms. The carbon chain in said alkyl group may be straight or branched or cyclic. Furthermore, said chain may contain a cyclic portion. Thus, as the alkyl group, there may be mentioned, among others, methyl, ethyl, n-propyl, isopropyl, cyclopropyl, n-butyl, tert-butyl, sec-butyl, n-pentyl, 1-ethylpropyl, isopentyl and benzyl.

In the antitumor composition according to the invention, the active ingredient compound may be in its free form or in the form of a pharmacologically acceptable nontoxic salt, for example an alkali metal salt, such as a sodium salt or potassium salt, or an alkaline earth metal salt, such as a calcium salt or magnesium salt. In cases where said compound is in a salt form, the carboxyl groups occurring in said compound may be either fully or partially involved in salt formation. Any of the possible salts can be used for preparing the composition according to the invention.

The antitumor composition according to the invention may contain one of or a combination of two or more of the active ingredient compounds defined hereinabove, depending on the intended purpose and/or necessity.

The active ingredient compounds to be used in the antitumor composition according to the invention can be produced by various means, for example in the following manner. Thus, S-(α ,β -dicarboxyethyl)glutathione

can be extracted, isolated and purified from yeasts or the bovine crystalline lens, for instance, since it occurs there. Alternatively, S-(α , β -dicarboxyethyl)glutathione can be synthesized by allowing an equimolar mixture of the starting materials glutathione and maleic acid in aqueous solution or in alcoholic aqueous solution to stand with warming or at room temperature for 1 to 2 days. The use of a maleic acid monoester or diester in lieu of maleic acid gives the corresponding S-(α ,β -dicarboxyethyl)glutathione ester derivative. While the compounds synthesized in the above manner have a newly introduced asymmetric carbon atom in their molecules and hence involve optical isomers, both isomers as well as mixtures thereof can suitably be used in the practice of the invention.

The active compound to be used in the antitumor composition of the present invention is a substance present in the body or an ester derivative thereof and therefore it is clear to be scarcely toxic, as evidenced by the data generated and shown in Test Example 2 which is described herein, hence is excellent in safety and can be advantageously used in various dosage forms against various malignant tumors.

As the malignant tumors which can be treated with the composition according to the invention, there may be mentioned, for instance, solid cancers, such as gastric cancer, hepatic cancer, rectal cancer and pulmonary cancer, and blood cancers, such as leukemia and Hodgkin's disease.

In the treatment of malignant tumors, such as those mentioned above, the composition according to the invention is suitably applied either orally or parenterally depending on the disease to be treated or on systemic conditions of patients. Usable dosage forms are, for instance, solid form preparations, such as tablets, granules, powders, capsules and suppositories, and liquid form preparations such as syrups, elixirs and injections. These preparations can be prepared by methods well known in the art. The preparations may contain, as necessary, those inert components, auxiliaries and additives that are commonly used in the pharmaceutical practice, for example excipients, diluents, binders, wetting agents, humectants, disintegrants, lubricants, dispersants, corrigents, flavors, buffers, surfactants, solubilizers, preservatives, isotonizing agents, stabilizers and pH adjusting agents, each in an appropriate amount.

The dose of the active ingredient compound mentioned above may vary depending on the compound, patient's age and body weight, dosage form, symptom to be treated and other factors. Generally, however, it is recommendable that, in the case of an injection, for instance, about 10 to 500 mg be administered once daily to each adult human; in the case of peroral dosage forms, about 10 to 1000 mg be administered several times daily to each adult; and, in the case of suppositories, about 10 to 500 mg be administered once daily to each adult.

The composition according to the invention may further contain another or other antitumor agents and/or another or other agents capable of producing other pharmacological effects unless the object of the invention becomes unattainable.

The following examples are further illustrative of the present invention.

Test Example 1

Pharmacological activity testing in mice with sarcoma 180

Sarcoma 180 cells (1.16 X $10^7$ cells/animal) were transplanted into groups of 8 ddy strain male mice (5 to 6 weeks of age) subcutaneously at the back on the right side of the body. In one group, the compound S- (α ,β -dicarboxyethyl)glutathione (hereinafter referred to as "DCE-GS" for short) in the forms of a 1.0% (w/v) injectable solution (pH 6.5; isotonized with sodium chloride) was intraperitoneally administered to the animals in a volume corresponding to the dose of 100 mg/kg once daily for 11 days. The control group was intraperitoneally given the same volume of physiological saline once daily for the same period. On day 12, the animals were sacrificed, the tumor was excised from each animal and weighed. The results thus obtained are shown below in Table I.

Table I Tumor weights (g)

| Individual No. | Physiological saline group | DCE-GS group |
|---|---|---|
| 1 | 4.82 | 2.48 |
| 2 | 3.33 | 1.92 |
| 3 | 2.48 | 2.09 |
| 4 | 2.95 | 1.47 |
| 5 | 4.04 | 1.95 |
| 6 | 2.40 | 1.11 |
| 7 | 1.95 | 1.62 |
| 8 | 5.63 | 1.69 |
| Mean | 3.45± 0.42 | 1.79± 0.14 |

$$\text{Tumor growth inhibition} = \frac{3.45 - 1.79}{3.45} \times 100 = 48.1\ \%$$

The above results clearly indicate that the compound tested has marked antitumor activity.

Test Example 2

Acute toxicity testing by intravenous administration

Acute toxicity testing was carried out by intravenous administration of DCE-GS to groups of 5 male ddy strain mice weighing about 20 g. The doses used were 100, 200, 400, 800 and 1,600 mg/kg (common ratio = 2). The injection were adjusted to pH 7 with 1 N sodium hydroxide. Neither deaths nor behavioral abnormalities were noted during 72 hours of observation.

Synthesis Example 1

S-(α ,β -Dicarboxyethyl)glutathione

Glutathione (9.2 g) and 5.0 g of maleic acid are dissolved in 150 ml of water and the solution is allowed to stand at room temperature for 12 hours. The reaction mixture is sampled (one or two drops) and one drop of 0.01 N $I_2$ test solution is added to the sample. After confirming, in this manner, that there is no more iodine consumption, 6.6 g of copper acetate (monohydrate) is added to the reaction mixture. If a precipitate, which

is small in amount, is found, the precipitate is filtered off. The filtrate is concentrated to about 70 ml, ethanol is added to the concentrate, and the resulting blue copper salt precipitate is collected by filtration. This is recrystallized from water-ethanol. This copper salt is further dissolved in 200 ml of water, hydrogen sulfide is passed through the solution to cause precipitation of copper sulfide, and the precipitate is filtered off. The filtrate is concentrated under reduced pressure and ethanol is added to the residue, whereupon white crystals appear. The crystals are collected by filtration, washed with ethanol and recrystallized from water-ethanol to give about 9 g of the desired compound as white amorphous crystals (hygroscopic).

Synthesis Example 2

Sodium salt of S-(α ,β -dicarboxyethyl)glutathione

S-(α , β -Dicarboxyethyl)glutathione (2 g ) is dissolved in 40 ml of water, the solution is adjusted to pH 7 with 1 N NaOH and then concentrated under reduced pressure at a temperature not exceeding 30 °C. Ethanol is added to the concentrate and the resulting white crystals are collected by filtration and recrystallized from water-ethanol to give 2.1 g of the desired compound as a white crystalline powder.

Synthesis Example 3

Calcium salt of S-(α , β -dicarboxyethyl)glutathione

5-(α , β -Dicarboxyethyl)glutathione (2 g) is dissolved in 40 ml of water, 1 g of calcium carbonate is added to the solution, and the mixture is stirred with warming. When carbon dioxide gas evolution is no more observed, the excess of calcium is filtered off. The filtrate is concentrated under reduced pressure. Ethanol is added to the concentrate and the resulting white crystalline precipitate is collected by filtration and recrystallized from water-ethanol to give 2.2 g of the desired compound as a white crystalline powder.

Synthesis Example 4

Magnesium salt of S-(α , β -dicarboxyethyl)glutathione

S-(α , β -Dicarboxyethyl)glutathione (2 g) is treated with 1 g of basic magnesium carbonate in the same manner as mentioned above for the production of the calcium salt, to give 2.2 g of the magnesium salt.

Synthesis Example 5

Sodium salt of S-(diethyl-α , β -dicarboxyethyl) glutathione

Glutathione (9.2 g) and 5.6 g of diethyl maleate are dissolved in 150 ml of 30% (v/v) ethanol. The solution is adjusted to pH 6 with 2 N sodium hydroxide and then stirred at 50°C for about 5 hours. The reaction mixture is sampled (two drops) and one drop of 0.01 N iodine test solution is added to the sample. When the color of iodine does not fade any more, gaseous hydrogen sulfide is passed through the reaction mixture. The mixture is allowed to stand overnight and then concentrated, whereby the hydrogen sulfide gas is distilled off. Water (150 ml) is added to the residue for dissolution of the residue. Copper acetate monohydrate (6.6 g) is added to and dissolved in the solution. The copper salt gradually precipitates out. The precipitate is collected by filtration, washed with water and suspended in 150 ml of water. Gaseous hydrogen sulfide is passed through the suspension with stirring for the formulation of copper sulfide. The copper sulfide is filtered off, the filtrate is concentrated, ethanol (200 ml) was added to the residue for dissolving the same, and the solution is adjusted to pH 6 by gradually adding an ethanolic sodium hydroxide solution, whereupon white crystals precipitate out. These are collected by filtration, washed with ethanol and dissolved in water for recrystallization. The solution is concentrated as far as possible and then ethanol is added. The resulting crystalline precipitate is collected by filtration and dried to give 8.5 g of S-diethyl-α , β -dicarboxyethyl)glutathione sodium salt. TLC on silica gel: Rf = 0.28 (n-butanol-acetic acid-water = 4:1:1).

Synthesis Example 6

Sodium salt of S-di-n-butyl-α , β -dicarboxyethyl) glutathione

Glutathione (9.2 g) and 7.5 g of di-n-butyl maleate are dissolved in 150 ml of 50% (v/v) ethanol and the reaction is carried out in the same manner as Synthesis Example 5. The solvent is then distilled off and the residue is dissolved in 150 ml of water. Addition of 200 ml of 3.3% aqueous copper acetate to the solution results in precipitation of the water-insoluble copper salt. The precipitate is collected by filtration, washed with water and suspended in 300 ml of 50% (v/v) ethanol. Hydrogen sulfide is passed through the suspension with stirring for the formation of copper sulfide. The copper sulfide is filtered off, the filtrate is concentrated for the removal of hydrogen sulfide. The concentrate is dissolved again in 150 ml of 50% (v/v) ethanol, and the solution is adjusted to a pH of about 6 by addition of 2 N sodium hydroxide solution and then concentrated. Ethanol, acetone and isopropyl ether are added to the concentrate. The resulting white crystalline precipitate is collected by filtration, washed with acetone and dried to give 9.7 g of S-(di-n-butyl-α ,β -dicarboxyethyl)glutathione sodium salt as hygroscopic crystals. TLC on silica gel: Rf = 0.40 (n-butanol-acetic acid-water = 4:1:1).

Synthesis Example 7

Calcium salt of S-(di-n-butyl-α , β -dicarboxyethyl)glutathione

The procedure of Synthesis Example 6 was followed using calcium carbonate in lieu of the 2 N sodium hydroxide. Addition of acetone to the concentration residue gives white crystals. They are recrystallized from ethanol-acetone to give 7.5 g of S-(di-n-butyl-α ,β -dicarboxyethyl)glutathione calcium salt.

Synthesis Example 8

Sodium salt of S-(monoethyl-α , β -dicarboxyethyl)glutathione

Glutathione (9.2 g) and 4.5 g of monoethyl maleate are dissolved in 150 ml of water, the solution is adjusted to pH 6.0 with 2 N sodium hydroxide, and the reaction is carried out in the same manner as Synthesis Example 5. The reaction mixture is concentrated, ethanol is added to the residue, and the resulting precipitate white crystals are collected by filtration and dissolved in water for recrystallization. The aqueous solution is concentrated and ethanol is added to cause crystallization. Yield 8.0 g. TLC on silica gel: Rf = 0.17 (n-butanol-acetic acid-water = 4:1:1).

Dosage Form Example 1

Peroral tablets

| | |
|---|---|
| DCE-GS calcium salt | 100 mg |
| Lactose | 80 mg |
| Starch | 17 mg |
| Magnesium stearate | 3 mg |

Tablets are produced by a conventional method using the above materials, the quantities given above being for one tablet. The tablets may be sugar-coated as necessary.

Dosage Form Example 2

Injectable solution

| | |
|---|---|
| DCE-GS sodium salt | 1.5 mg |
| Sodium chloride | 0.6 mg |
| Distilled water for injection | 100 ml |

The above ingredients are mixed up and sterilized by bacterial filtration. The filtrate is distributed in 2-ml portions into glass ampoules and the ampoules are sealed.

Dosage Form Example 3

Suppositories

| | |
|---|---|
| DCE-GS sodium salt | 2 g |
| Cacao butter | 20 g |

Suppositories are produced by a conventional method using the above materials, the quantities being given for 10 suppositories.

## Claims

1. Use of a compound of the formula

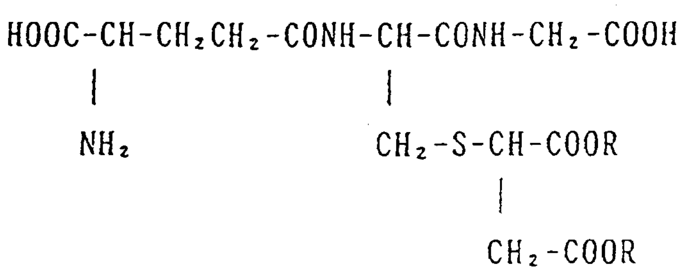

wherein R's are the same or different and each means a hydrogen atom or a lower alkyl group having 1-10 carbon atoms or a pharmaceutically acceptable salt thereof as an active ingredient for preparing a medicament for fighting hepatic cancer.

2. The use according to claim 1, wherein the medicament is in the form of tablets, granules, powders, capsules, suppositories, syrups, elixirs or injections.

## Patentansprüche

1. Verwendung einer Verbindung der Formel

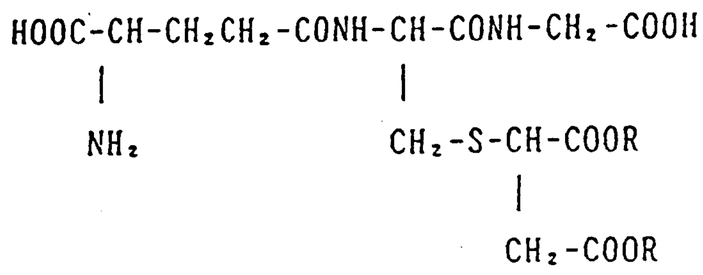

worin die R gleich oder verschieden sind und jedes ein Wasserstoffatom oder eine Niederalkylgruppe mit 1 bis 10 Kohlenstoffatomen bedeutet oder ein pharmazeutisch annehmbares Salz davon, als ein Wirkstoff zur Herstellung eines Medikaments zur Bekämpfung von Lebercarcinomen.

**2.** Verwendung nach Anspruch 1, worin das Medikament in Form von Tabletten, Granula, Pulvern, kapseln, Zäpfchen, Sirups, Elexieren oder Injektionen vorliegt.

## Revendications

**1.** Utilisation d'un composé de formule

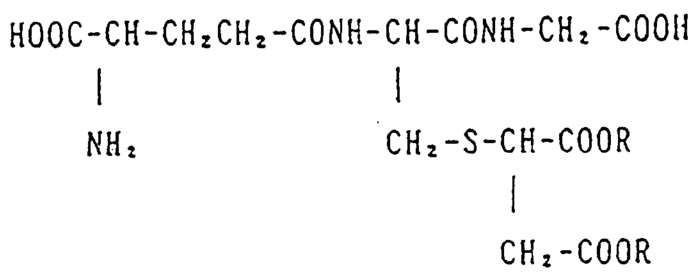

dans laquelle les radicaux R sont identiques ou différents et représentent chacun un atome d'hydrogène ou un groupe alkyle inférieur ayant de 1 à 10 atomes de carbone, ou d'un de ses sels pharmaceutiquement acceptables, en tant que principe actif pour préparer un médicament destiné à lutter contre le cancer du foie.

**2.** Utilisation selon la revendication 1, dans laquelle le médicament se présente sous la forme de comprimés, de granules, de poudres, de gélules, de suppositoires, de sirops, d'élixirs ou de solutions injectables.